# EUROPEAN PATENT APPLICATION

(11) **EP 3 889 966 A1**
(43) Date of publication of application: **06.10.2021**
(21) Application number: 20167655.8
(22) Date of filing: 02.04.2020
(51) Int. Cl.: G16H 20/40

(54) **ABLATION ZONE ASSESSMENT AND CONFIGURATION**

(71) Applicant: Koninklijke Philips N.V., 5656 AG Eindhoven (NL)
(72) Inventor: HAUVAST, Guillaume Leopold Theodorus Frederik, 5656 AE Eindhoven (NL)
(74) Representative: Philips Intellectual Property & Standards

(57) **Abstract**

The invention presents assessing and improving the quality of a predicted ablation zone generated from data obtained during an ablation treatment. In particular, the present invention proposes to compare an ablation zone generated during an ablation treatment to an ablation zone generated after a treatment has taken place.

## Description

### FIELD OF THE INVENTION

The present invention relates to the field of ablation devices, and in particular to ablation devices that capture intra-treatment ablation data.

### BACKGROUND OF THE INVENTION

Percutaneous thermal ablation is an interventional cancer treatment for a subject that has seen a significant increase in adoption in the past decade. Thermal ablation can be delivered using various ablation modalities, including radiofrequency (RF), microwave (MW), high-intensity focused ultrasound (HIFU), focal laser ablation (FLA), irreversible electroporation (IRE), cryo-ablation, etc.

In clinical practice, these ablation procedures consist of placing one or more ablation applicators of an ablation system inside or near a target region with the help of image guidance. Typically, physicians place these needle-like applicators while inspecting real-time ultrasound or interventional radiology images (such as CT or MR images), based on information provided from the manufacturer, results in clinical trials and personal experience. In particular, these images are used to help the clinician place the ablation applicators at the correct/desired region.

The use of more advanced ablation therapy planning systems (ATPS) that provide feedback on target coverage during the procedure is being investigated. These systems are able to reconstruct the overall ablation zone based on the anticipated ablation zone around an individual ablation (an "individual ablation zone"). Information on the shape and dimensions of such an individual ablation zone is typically provided by the manufacturer of the ablation device. Information on the overall ablation zone can aid a clinician during an ablation procedure to help them fully ablate a desired area or zone of the patient.

After an ablation treatment has been performed, it is somewhat common for a subject to undergo an MRI exam to accurately establish an ablated zone of the subject, i.e. determine a "true" ablation zone. The results of the MRI exam can be used to guide future ablations for the subject, for example, so that any areas missed during an earlier ablation are ablated during a subsequent ablation treatment.

### SUMMARY OF THE INVENTION

The invention is defined by the claims.

According to examples in accordance with an aspect of the invention, there is provided a computer-implemented method for enabling assessment of the accuracy of a predicted ablation zone obtained from intra-treatment data generated by an ablation system during an ablation treatment.

The computer-implemented method comprises: obtaining intra-treatment ablation data generated by an ablation system during an ablation treatment, the intra-treatment ablation data providing information on an ablation treatment performed on a subject; processing the intra-treatment ablation data to construct a predicted ablation zone that predicts the ablation zone produced by the ablation treatment; obtaining post-treatment ablation data generated after the ablation treatment is performed on the subject, the post-treatment ablation data providing information on the achieved ablation zone of the subject; processing the post-treatment ablation data to construct the achieved ablation zone; and registering the predicted ablation zone and the achieved ablation zone with respect to one another to thereby enable assessment of the accuracy of a predicted ablation zone.

The present invention relies upon the recognition that the information on the dimensions and shape of an individual ablation zone (e.g. provided by a manufacturer of an ablation device) are inaccurate. This is because such dimensions and/or shapes are typically established under experimental conditions (e.g. ex-vivo, in animals or in different tissues) that do not match with actual use.

The inventors have therefore recognized a need and/or desire for enabling the accuracy of a predicted ablation zone (when an ablation plan is executed) to be assessed. The inventors propose to enable such an assessment to take place by registering an (achieved) ablation zone derived from post-treatment ablation data with respect to an (predicted) ablation zone derived from intra-treatment ablation data, e.g. so that a comparison between an achieved (i.e. "true") ablation zone and a predicted ablation zone can be performed.

The present invention thereby provides a new mechanism by which an accuracy, e.g. prediction error, of a predicted ablation zone is able to be determined or calculated.

At least one embodiment of the invention comprises determining an error between the predicted ablation zone and the achieved ablation zone. In other words, embodiments may comprise actively determining a prediction error of the predicted ablation zone. This prediction error may, for example, be presented, displayed or otherwise made available (to a clinician or operator) to aid their understanding, e.g. for the purposes of quality assurance, guiding subsequent treatment options and the like.

The intra-treatment ablation data may comprise applicator information on the position, orientation and/or type of one or more ablation applicators during the ablation treatment. In such an embodiments, the step of constructing a predicted ablation zone may comprise processing the applicator information to construct the predicted ablation zone. In particular, the applicator information can be processed to construct an anticipated individual ablation zone around each individual applicator, which can thereby together form the overall anticipated ablation zone.

In some embodiments, the step of processing the intra-treatment ablation data may comprise using ablation application settings to construct the predicted ablation zone. The applicator settings may define the anticipated individual ablation zone around an individual ablation performed by each ablation applicator. In particular, the ablation application settings may allow a mapping between applicator position, orientation and/or type (e.g. provided by applicator information) and an individual ablation zone to take place.

The method may further comprise determining an error between the predicted ablation zone and the achieved ablation zone. In other words, the difference between the predicted ablation zone and the achieved ablation zone may be quantified through use of an error calculation. The formatting of the determined error is preferably numeric, e.g. a root means squared error. Suitable examples of determining an error between ablation zones would be apparent to the skilled person.

The precise data format of the ablation zones can differ depending upon implementation details. For example, an ablation zone may be formed as (or comprise) a mesh, a contour set, a binary mask, one or more analytical description(s) and so on. An ablation zone could be embedded in a communication standard, such as DICOM or DICOM RT.

In particular, the data format of an ablation zone should enable a 3D construction of the ablation zone to take place, e.g. defining a 3D position, shape, orientation and/or one or more other properties that enable a 3D construction of the ablation zone to take place. Other methods of defining an ablation zone would be apparent to the skilled person.

The method may further comprise a step of adjusting one or more settings of the ablation system based on the determined error. In particular, the adjusted setting(s) may comprise one or more settings that control the generation of the predicted ablation zone based upon the intra-treatment ablation data.

Put another way, subsequent steps of generating the predicted ablation zone may comprise processing the intra-treatment ablation data using one or more adjusted settings of the ablation system to generate the predicted ablation zone.

The adjustment to the settings can be automated or responsive to a user input. For example, the error may be displayed to a user/clinician/operator, who can use the error information to adjust how the intra-treatment ablation data is processed to construct the predicted ablation zone. For example, if the predicted ablation zone is constructed by combining individual ablation zones (for different instances of placement of an ablation applicator and/or different ablation applicators), then the default size and/or dimension of each individual ablation zone can be modified based on the determined error.

This process can be automated through the use of machine-learning methods, multi-dimensional optimization approaches and so on. By way of example, a multi-dimensional optimization approach may be used to automatically modify one or more settings in an effort to reduce a numerical error between a predicted ablation zone and an achieved ablation zone.

In some embodiments: the intra-treatment ablation data comprises applicator information identifying at least a position and type of one or more ablation applicators during the ablation treatment; the step of constructing a predicted ablation zone comprises processing the applicator information and one or more ablation applicator settings to construct the predicted ablation zone, the one or more applicator settings defining the anticipated ablation zone around an individual ablation performed by each ablation applicator based on the applicator information; and the step of adjusting one or more settings of the ablation system comprises adjusting at least one of the one or more applicator settings.

The ablation applicator information may identify a spatial position and/or orientation of the one or more ablation applicators during the ablation treatment. This enables the method to predict the size, dimensions and/or shape of each individual application of ablation, e.g. using applicator settings. Applicator settings may map between a position and type of an applicator and an anticipated individual ablation zone.

The method may further comprise a step of sharing the adjusted settings of the ablation system with one or more other ablation systems.

In one or more embodiments, the step of registering the predicted ablation zone and the achieved ablation zone comprises registering the intra-treatment ablation data and the post-treatment ablation data with respect to one another, to thereby register the predicted ablation zone and the achieved ablation zone with respect to one another.

Registering the intra- and post-treatment ablation data with respect to one another enables the ablation zones to be indirectly registered with one another.

The step of registering the intra-treatment ablation data and the post-treatment ablation data with respect to one another optionally comprises: obtaining reference ablation data; registering the intra-treatment ablation data to the reference ablation data; and registering the post-treatment ablation data to the reference ablation data, thereby indirectly registering the intra-treatment ablation data to the post-treatment ablation data.

Thus, the intra- and post-treatment ablation data may be registered with respect to some reference ablation data in order to facilitate the registration of the intra- and post-treatment ablation data with one another. This provides a method of registering two potential disparate pieces of data with one another.

Registering the intra-treatment ablation data with the reference ablation data enables the intra-treatment ablation data to be registered during the ablation treatment itself, e.g. avoiding a need to perform potentially complex registration or matching after registration has been performed.

In particular examples, during an ablation treatment, images generated for aiding a clinician in performing the ablation treatment can be used to register the intra-treatment ablation data with respect to the reference ablation data.

The reference ablation data may, for example, comprise pre-treatment ablation data, generated before the ablation treatment is performed on the subject.

In some embodiments, the step of registering the predicted ablation zone and the achieved ablation zone comprises directly processing the predicted ablation zone and the achieved ablation zone to register the two together.

In other words, the constructed ablation zones may be directly processed in order to register the two together, rather than being indirectly registered by registering the corresponding ablation data.

In some embodiments, the step of registering the predicted ablation zone and the achieved ablation zone may comprise registering the predicted ablation zone with a reference ablation zone and registering the achieved ablation zone to a reference ablation zone, thereby registering the two ablation zones together. The reference ablation zone may be a default or representative ablation zone, e.g. for a procedure undertaken when the executed ablation treatment is performed on a subject.

The post-treatment ablation data may comprise one or more medical images of the subject captured after the ablation treatment, the one or more medical images providing visual information on the achieved ablation zone.

The post-treatment ablation data may thereby comprise any suitable medical image of the subject that provides an image of the achieved ablation zone. A particularly preferable example of a medical image is an MR(I) image. An MR image provides the ability to delineate the achieved/true ablation zone based on the soft tissue contrast generated by the MR sequences.

The intra-treatment ablation data may comprise one or more medical images captured during the ablation treatment.

Suitable examples of some medical images include any suitable ultrasound or interventional radiology image (such as a CT or MR images).

In at least one embodiment, the post-treatment ablation data comprises one or more medical images of the subject captured after the ablation treatment, the one or more medical images providing visual information on the achieved ablation zone; and the step of registering the predicted ablation zone and the achieved ablation zone with respect to one another comprises registering at least one of the medical images captured during the ablation treatment against at least one of the medical images captured after the ablation treatment, to thereby register the predicted ablation zone and the achieved ablation zone with respect to one another.

Optionally, the step of registering at least one of the medical images captured during the ablation treatment against at least one of the medical images captured after the ablation treatment comprises: obtaining a reference medical image; registering at least one of the medical images captured during the ablation treatment against the reference medical image; registering at least one of the medical images captured after the ablation treatment against the reference medical image, to thereby indirectly register at least one of the medical images captured during the ablation treatment against at least one of the medical images captured after the medical image.

The method may further comprise a step of displaying the predicted ablation zone and the achieved ablation zone, wherein the display is based on the registration between the predicted ablation zone and the achieved ablation zone.

In particular, the predicted and achieved ablation zones can be displayed in a single frame of reference, which enables quality assurance of the ablation zones. Other methods of displaying ablation zones based on the registration between the two will be apparent to the skilled person.

According to another aspect of the invention, there is provided a computer program product comprising computer program code means which, when executed on a computing device having a processing system, cause the processing system to perform all of the steps of any herein described method.

According to another aspect of the invention, there is provided an ablation system for enabling assessment of the accuracy of a predicted ablation zone obtained from intra-treatment data generated by an ablation system during an ablation treatment.

The ablation zone system comprises an ablation therapy planning system (ATPS) and an ablation therapy follow-up system (ATFS).

The ablation therapy planning system is configured to: obtain intra-treatment ablation data generated by an ablation system during an ablation treatment, the intra-treatment ablation data providing information on an ablation treatment performed on a subj ect.

The ablation therapy follow-up system is configured to obtain post-treatment ablation data generated after the ablation treatment is performed on the subject, the post-treatment ablation data providing information on the achieved ablation zone of the subject; process the post-treatment ablation data to construct the achieved ablation zone; and register a predicted ablation zone, that predicts the ablation zone produced by the ablation treatment, and the achieved ablation zone with respect to one another to thereby enable assessment of the accuracy of a predicted ablation zone.

Either the ATPS or the ATFS is configured to process the intra-treatment ablation data to construct the predicted ablation zone.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an ablation zone system in which embodiments of the invention can be implemented;
Fig. 2 is a flowchart illustrating a method according to an embodiment of the invention;
Fig. 3 is a block diagram illustrating an ablation system according to an embodiment of the invention; and
Fig. 4 illustrates an MR image having a predicted and actual ablation zone superimposed thereon.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides concepts for assessing and improving the quality of a predicted ablation zone generated from data obtained during an ablation treatment. In particular, the present invention proposes to compare a predicted ablation zone generated during an ablation treatment to an actual ablation zone generated after a treatment has taken place.

Embodiments of the invention are based on the realization that predicted ablation zones can be inaccurate, e.g. due to the methods from which data used to predict an ablation zone are generated. This can lead to inaccurate or incomplete ablation during an ablation treatment, which would lead to reduced quality of patient treatment.

The invention can be employed in any clinical environment, for example, in a hospital in which tumor ablations take place.

In the context of the present invention, an "ablation zone" is data that provides a (3D) model of an ablated area, and would be well understood by the skilled person. In particular, the data forming an ablation zone may enable a (3D) visualization of an ablated area of a subject to be visually presented or displayed (e.g. to a user).

The data format of an ablation zone should enable a (3D) construction of the ablation zone to take place, e.g. defining a (3D) position, shape, orientation and/or one or more other properties that enable a (3D) construction of the ablation zone to take place. The precise data format of an ablation zone is not essential to achieving the underlying inventive concept of the present invention, provided that ablation zones can be (spatially) registered with respect to one another (and preferably be capable of being processed to determine an error between ablation zones).

Suitable examples of data formats for an ablation zone include a mesh, a contour set, a binary mask, one or more analytical description(s) and the like. An ablation zone could be embedded in a communication standard, such as DICOM or DICOM RT.

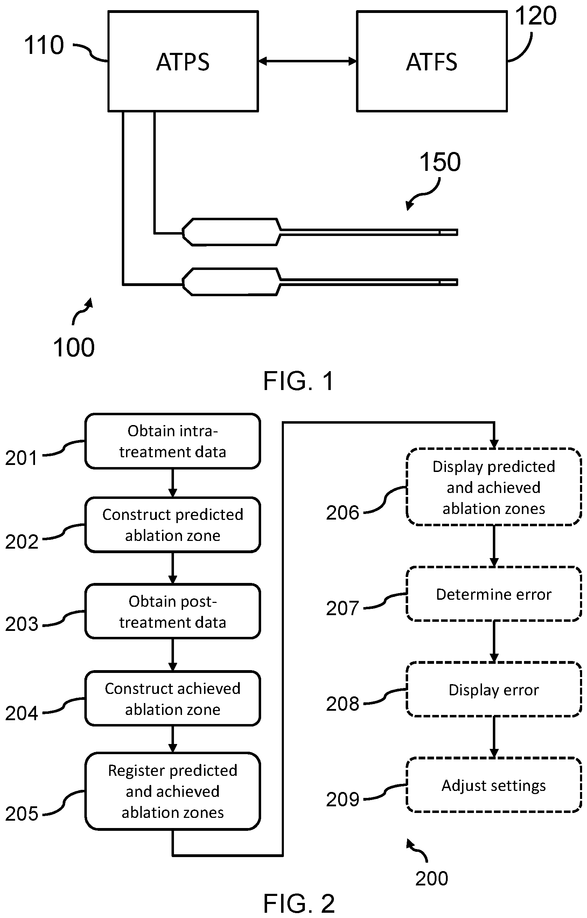

Figure 1 illustrates an ablation system 100 in which embodiments of the invention can be implemented.

The ablation system 100 comprise an ablation therapy planning system, ATPS, 110 and an ablation therapy follow-up system, ATFS 120. The illustrated ablation system 100 also comprises one or more ablation applicators 150, although these are not an essential part of the ablation system.

The ablation applicator(s) 150 are configured so that, when they are appropriately positioned with respect to a subject and activated, they deliver an individual ablation to an area of the subject. Typically, positioning of the ablation applicator(s) is performed manually by a clinician.

The size (e.g. dimensions) and shape of the individual ablation can depend upon a number of factors, such as the type of the applicator, a length of activation, a position of the applicator and so on. Different ablation applicators 150 may deliver ablation according to different modalities. For example, thermal ablation can be delivered using various ablation modalities, including radiofrequency (RF), microwave (MW), high-intensity focussed ultrasound (HIFU), focal laser ablation (FLA), irreversible electroporation (IRE), cryo-ablation, etc.

Of course, the ablation applicator(s) 150 may be controlled by an ablation controller, which may be integrated into the ablation therapy planning system or separate, that can control when ablation is performed by the ablation applicator. The ablation applicator may be activated in response to a user input (e.g. a user trigger) or automatically.

Typically, ablation of a subject is performed by following an ablation plan. An ablation plan may dictate the recommended positions for ablation applicator(s) 150 with respect to a subject in order to achieve ablation of a desired/planned ablation zone. The size/shape of the desired/planned ablation zone may be selected so as to ablate a desired area of the subject (e.g. a location of a tumor within the subject).

The ATPS 110 may guide a clinician during an ablation treatment. In particular, the ATPS may be configured to provide real-time information (e.g. real-time ultrasound or interventional radiology images (CT/MR)) that aid a clinician in positioning the applicator(s) within the subject.

The ATPS 110 may be configured to act as an ablation controller, e.g. controlling when the one or more ablation applicators 150 activate to apply an ablation. Activation of an ablation applicator may be automatic (e.g. when an applicator has reached a recommended position) or responsive to a user input (activated by a clinician).

In some further examples, the ATPS may be configured to obtain or generate an ablation plan, which may identify suitable locations for the ablation applicator(s) in order to ablate a desired area of the subject (e.g. ablate a tumor or cancerous region). The ATPS may indicate to a clinician these desired locations (e.g. using markers on an image) or a distance of the applicator from a desired location.

The ATPS 110 is configured to record intra-treatment ablation data, e.g. data that is obtained during the ablation procedure.

In particular examples, the intra-treatment data may include information on the positions, location and/or orientation of the ablation applicator(s) when they perform an ablation. The intra-treatment data may also provide information on the type of ablation performed (e.g. a type or mode of the ablation applicator when it performs an ablation).

In other examples, the intra-treatment data may provide information on a path taken by an ablation applicator during an ablation treatment.

In some further examples, the intra-treatment data may comprise intra-treatment medical images, e.g. CT, ultrasound or MR images of the area under ablation.

The ATPS 110 may be configured to process the intra-treatment ablation data to construct a predicted ablation zone that predicts the (overall) ablation zone produced by the ablation treatment. This process can be performed during or after the treatment. If performed during the treatment, the predicted ablation zone may be periodically updated (and displayed to a user) to aid a clinician during the ablation treatment, e.g. to identify the predicted areas which have been ablated so far.

Various embodiments for this process would be apparent to the skilled person, and may depend upon the implementation details for the intra-treatment ablation data.

In one embodiment, where the intra-treatment ablation data provides information on the positions, location and/or orientation of the ablation applicator(s) when they perform an ablation, the ATPS may be configured to predict the size, shape and position of each individual ablation zone generated by an ablation. Other information about the ablation applicator may also be used, e.g. the type of applicator and/or the mode of the applicator. It will be understood that an ablation applicator, when performing an ablation, ablates an individual ablation zone.

To generate an individual ablation zone, the ATPS 110 may use ablation settings that define the size and/or shape of the individual ablation, e.g. based on the type, mode, location and/or orientation of the ablation applicator when it is activated (i.e. performs an ablation). The settings may, for example, define a size of a major and minor axes (for an elliptical ablation), as well as the offset of a particular ablation zone with respect to the applicator.

The ATPS 110 can then construct the predicted (overall) ablation zone (hereinafter "predicted ablation zone") by combining the individual ablation zone(s). In other words, the ATPS can predict which area of the subject is ablated.

Thus, the ATPS 110 may be able to construct a predicted ablation zone using intra-treatment ablation data. Other methods of constructing a predicted ablation zone using intra-treatment ablation data would be apparent to the skilled person.

Purely by way of another example, the ATPS may be configured to generate a predicted ablation zone from (medical) images captured during the ablation treatment procedure (e.g. using a machine-learning algorithm or image segmentation process).

By way of yet another example, the ATPS may be configured to generate a predicted ablation zone from a path taken by the ablation applicator(s) during the ablation procedure, e.g. by comparing the path taken with historic paths and the corresponding ablation zones generated for the historic paths.

By way of yet another example, where the intra-treatment ablation data provides information on the positions, location and/or orientation of the ablation applicator(s) when they perform an ablation, the ATPS may be configured to directly map between the identified positions, locations, and/or orientation of the ablation applicator(s) and a predicted ablation zone (e.g. by referencing a look-up table), rather than by predicting and combining individual ablation zones.

From the foregoing, it will be apparent that the predicted ablation zone is derived from the intra-treatment ablation data, i.e. is intra-treatment derived data.

In some examples, the ATPS may be adapted to dynamically generate and display (at a user interface) a predicted ablation zone during the ablation treatment, e.g. based on live intra-treatment ablation data generated by the ATPS. This may be iteratively performed throughout the ablation treatment, to thereby update the predicted ablation zone throughout the ablation treatment. This enables a user/clinician to track the predicted progress of the overall ablation.

In other examples, the predicted ablation zone may be generated once treatment is complete. The user may use instead simply use medical images (and optionally recommended locations for the applicator(s)) when controlling the treatment.

The ATFS 120 is configured to generate an achieved ablation zone, identifying the "true" or measured size/shape of the ablation zone. The ATFS is operated after the ablation treatment has been completed (e.g. between 3-21 days after the ablation treatment), and is usable to determine where further ablation is required (e.g. to eliminate a tumor).

The achieved ablation zone is determined from post-treatment ablation data, i.e. data obtained after ablation treatment has been completed. Suitable examples of post-treatment ablation data include medical image data, e.g. an MR (magnetic resonance) image, i.e. "post-treatment medical images".

An ATFS offers the ability to delineate the "true" or achieved ablation zone based on the soft tissue contrast generated by the MR sequence. Methods of determining a true ablation zone using an ATFS would be apparent to the skilled person. Typically, such methods include segmenting one or more medical (e.g. MR) images using a machine-learning or segmentation approach, to identify the location of an ablated zone.

For the sake of completeness, it is noted that the generation of the achieved ablation zone from one or more medical images (forming the post-treatment ablation data) could be delivered using one or more of: manual delineation tools in 2D (e.g. contouring) or 3D (e.g. brush); semi-automatic delineation tools in 2D (e.g. active contours) or 3D (e.g. image adaptive brush); and/or automatic delineation tools using techniques ranging from classic, binary morphology to deep learning approaches (e.g. convolutional neural networks).

From the foregoing, it will be apparent that the achieved ablation zone is derived from the post-treatment ablation data, i.e. is post-treatment derived data.

In some examples, the ATFS may perform the steps of generating the predicted ablation zone from the intra-treatment ablation data. This may be performed using the previously described approach.

The present invention recognizes that the true/achieved ablation zone may differ from the predicted ablation zone. This can lead to inaccurate assumptions during an ablation treatment. For example, the predicted ablation zone may indicate that an entire desired region has been ablated, when in reality it may not have been (although this can only be recognized post-treatment).

However, the inventors have also recognized that it is highly difficult to accurately generate a "true" ablation zone during the ablation treatment, e.g. using medical images generated during an ablation treatment. This is because, during treatment, secondary effects like edema or bleeding often clutter intra-treatment visualization of the true/achieved ablation zone.

For these reason, the inventors have recognized the importance of assessing and preferably improving the accuracy of a predicted ablation zone.

Fig. 2 is a flow-chart illustrating a computer-implemented method 200 according to an embodiment of the invention. The method 200 can be carried out by the ablation system 100 previously described with reference to Fig. 1.

The method 200 comprises a step 201 of obtaining intra-treatment ablation data generated by an ablation system during an ablation treatment.

The intra-treatment ablation data provides information on an ablation treatment performed on a subject, such as applicator information identifying at least a position and type of one or more ablation applicators during the ablation treatment. The intra-treatment ablation data optionally provides one or more medical images of the subject which are obtained during the ablation treatment, e.g. of the area undergoing ablation.

The method 200 may then move to a step 202 of processing the intra-treatment ablation data to construct a predicted ablation zone that predicts the ablation zone produced by the ablation treatment.

Step 202 comprises processing the intra-treatment ablation data to construct a predicted ablation zone that predicts the ablation zone produced by the ablation treatment. Methods of processing intra-treatment ablation data have been previously described with reference to Fig. 1, although other methods of deriving the predicted ablation zone would be apparent to the skilled person.

In some embodiments, step 202 may comprise delineating or identifying regions present in the intra-treatment ablation data, e.g. by performing a segmentation process on the intra-treatment ablation data. By way of example, the intra-treatment ablation data may comprise one or more medical images captured during the ablation treatment, which can be delineated to identify regions (e.g. organs, bones or the like) of the subject.

Steps 201 and 202 may be performed by an ATPS, such as that described with reference to Fig. 1.

The method 200 then moves to a step 203 of obtaining post-treatment ablation data generated after the ablation treatment is performed on the subject. The post-treatment ablation data provides information on the achieved ablation zone of the subject, such as in the form of one or more medical images, such as any suitable ultrasound or interventional radiology image (e.g. a CT, ultrasound or MR image).

The method then moves to a step 204 of processing the post-treatment ablation data to construct the achieved ablation zone. Methods of constructing an achieved ablation zone from post-treatment ablation data would be apparent to the skilled person, e.g. segmenting or delineating one or more (medical) images of the post-treatment ablation data. Some methods of constructing an achieved ablation zone from post-treatment ablation data have been described with reference to Fig. 1.

In particular embodiments, step 204 comprises identifying delineating regions present in the post-treatment ablation data. By way of example, the post-treatment ablation data may comprise one or more medical images captured after the ablation treatment, which can be delineated to identify or delineate regions (e.g. organs, bones or the like) of the subj ect.

The method then moves to a step 205 of registering the predicted ablation zone and the achieved ablation zone with respect to one another to thereby enable assessment of the accuracy of a predicted ablation zone.

Various methods of registering the predicted ablation zone and the achieved ablation zone with respect to one another are contemplated in the present disclosure.

In some embodiments, the registration of the predicted ablation zone and the achieved ablation zone can be performed by registering the intra-treatment ablation data and the post-treatment ablation data with respect to one another (thereby registering the ablation zones derived therefrom together as well). The registration can be preserved when converting the intra- or post-treatment ablative data into ablative zones.

In these embodiments, the registration of the predicted ablation zone and the achieved ablation zone may effectively take place before the ablation zones need to be generated.

In other embodiments, the ablation zones are registered by processing the ablation zones themselves.

In the context of the present disclosure, the term "register" is used to mean "spatially register", e.g. so that a relative location, orientation and/or distance between different elements of different datasets or data instances can be identified. A number of methods of registering data with respect to one another are herein described, but other embodiments would be apparent to the skilled person.

By way of example only, if intra-treatment ablation data provides information on the location of ablation applicators during an ablation treatment, and post-treatment ablation data comprises one or more medical images of the ablated area, registering the intra-treatment ablation data and the post-treatment ablation data may comprise determining a relative location of the ablation applicators during the ablation treatment with respect to the medical image(s) of the ablated area.

The process for registering between the intra-treatment ablation data and the post-treatment ablation data may be performed manually or in an automatic manner.

If a manual approach is used, a visual representation of the intra-treatment ablation data (e.g. intra-treatment medical images) and/or the predicted ablation zone, as well as a visual representation of the post-treatment data (e.g. post-treatment medical images) and/or the achieved ablation zone, can be presented to the user/clinician via a user interface. The user/clinician may be able to manipulate the visual representation, via a user interface, in order to manually align or register the intra- or post-treatment (derived) data with respect to one another, to thereby register the predicted and achieved ablations zones with respect to one another.

For example, manual registration tooling may comprise providing pan and rotate tools to align visual representations of the predicted ablation zone and the achieved ablation zone, to enable a user to register the two ablation zones with respect to one another.

After registration has taken place, movement of one ablation zone may cause a corresponding movement in another ablation zone (e.g. to enable a user to manipulate the images and more clearly understand the difference between an actual and predicted ablation zone).

In another example, where delineation of regions within the intra- and post-treatment data has been performed, manual registration tooling may provide pan and rotate tools to allow a clinician to align delineated regions derived from of the intra- and post-treatment ablation data together, to thereby enable a user to register the intra- and post-treatment ablation data with respect to one another (e.g. align different organs and/or bones provided in the intra- and post-treatment delineations).

In yet another example, where intra- and post-treatment medical images are available, a toolset (e.g. providing pan, zoom and/or rotate tools) may be provided to enable a user to manually align one or more intra- and post-treatment medical image(s) with respect to one another, to enable the medical images to be registered with one another.

Rather than using a manual approach, any suitable automated approach could be used to register the intra- and post- ablative data with respect to one another.

By way of example only, where intra- and post-treatment medical images are available, image-based registration between intra-treatment and post- treatment medical images could be performed, e.g. using a maximization of cross correlation or mutual information.

Similarly, where delineation of regions within the intra- and post-treatment data has been performed, contour-based registration between delineated regions present in the intra- treatment and post-treatment data, e.g. using an iterative-closest-point algorithm to minimize the RMS error between delineated regions.

In another example, a contour-based registration between a predicted and achieved ablation zone could be performed, e.g. using an iterative-closest-point algorithm to minimize the RMS error between both.

The skilled person would appreciate that registering the intra-treatment ablation data and the post-treatment ablation together may comprise modifying one or more of the intra- and post-treatment ablation data, e.g. to account for any rotation, skew, mirroring, resizing performed to register the images together.

In such examples, if two aspects of the intra- and post-treatment data are registered with respect to one another (e.g. medical images are registered together), the other aspects of the intra- and/or post-treatment data and/or the derived ablation zones may be automatically modified based on the registration between the intra- and post-treatment data.

For example, if the intra- and post-treatment data both comprise one or more medical images, registering the intra- and post-treatment data together may result in the post-treatment medical images being modified (e.g. rotated or resized) to map onto the intra-treatment medical images (or vice versa). The predicted and/or achieved ablation zone may be appropriately modified to match or mirror the modification made to the corresponding intra- or post-treatment data to register the predicted and ablation zones together.

In the above-described embodiments, inter-treatment (derived) data and post-treatment (derived) data are registered directly with one another. However, in other examples, the predicted ablation zone and the achieved ablation zone may be indirectly registered via reference ablation data.

The reference ablation data may, for example, comprise pre-treatment ablation data, generated before the ablation treatment is performed on the subject.

By way of example only, pre-treatment ablation data may comprise one or more medical images of an area to undergo ablation, i.e. a "pre-treatment medical image". The intra-treatment ablation data may be used to register the predicted ablation zone with respect to the pre-treatment medical image (e.g. by registering an intra-treatment medical image with a pre-treatment medical image using any suitable image registration approach). Similarly, the post-treatment ablation data may be registered with respect to the pre-treatment medical image in a similar manner. In this way, the intra-treatment ablation data and the post-treatment ablation data can be indirectly registered with respect to one another.

Using reference ablation data enables the intra-treatment ablation data (e.g. medical images) to be used to register the predicted ablation zone to the achieved ablation zone, without the intra-treatment ablation data needing to be available at the same time as the post-treatment ablation data and/or the achieved ablation zone. In particular, by registering the predicted ablation zone with respect reference ablation data (e.g. a pre-operative image), the intra-treatment ablation data can then be discarded or deleted.

Thus, in a modification to the method 200, step 205 may be divided into a first sub-step of registering the predicted ablation zone to reference ablation data (e.g. using the intra-treatment ablation data) and a second sub-step of registering the achieved ablation zone to the reference ablation data (e.g. using the post-treatment ablation data). The first sub-step can be performed before steps 203 and 204 are performed, so that the intra-treatment data can be deleted (in an unseen step) before steps 203 and 204 are performed.

Whilst the above-described approach uses image registration techniques, other methods of registering data together will be apparent to the skilled person.

From the foregoing, it will be apparent that a number of methods, both direct and indirect, for (spatially) registering the predicted and achieved ablation zones together are envisaged.

Registering the predicted and achieved ablation zones with respect to one another enables an accuracy assessment of the predicted ablation zone to take place. This can be used for the purposes of quality assurance, e.g. to ensure that the prediction is correct, or may be used to correct and/or calibrating the information used to generate the predicted ablation zone.

An optional step 206 may be performed. Step 206 comprises, based on the obtained registration between intra- and post-treatment ablation zones data, displaying the predicted and achieved ablation zone (e.g. in a single frame of reference). This enables quality assurance to take place, e.g. to enable a user to assess the accuracy of the predicted ablation zone.

In some preferred embodiments, step 206 comprises displaying the predicted and/or achieved ablation zones with respect to the intra-treatment medical image(s) (if available) and/or the post-treatment medical image(s) (if available). The predicted and/or achieved ablation zones may overlay one or more of these images. The precise display may be configured to be responsive to a user input, e.g. a user can select whether and which medical images are used.

This process may require the predicted and achieved ablation zones to be registered with respect to the intra-treatment medical image(s) and/or the post-treatment medical image(s).

The method 200 may further comprise a step 207 of determining an error between the predicted ablation zone and the achieved ablation zone. In particular, step 207 may comprise calculating a numerical error between the predicted ablation zone and the achieved ablation zone.

The precise mechanism for determining an error may differ depending upon implementation details. In some examples, a root mean squared error or mean absolute error can be determined between the predicted ablation zone and the achieved ablation zone.

The method 200 may comprise a step 208 of displaying the determined error, e.g. at a display system. Step 208 is optional, but helps a clinician to understand and assess the accuracy of the predicted ablation zone.

In some embodiments, the method 200 comprises a step 209 of adjusting one or more settings of the ablation system. Preferably, this step is performed using an error determined in step 207. In particular, the one or more settings may be those used in the generation of the predicted ablation zone.

By way of example only, where the ablation system combines (predicted) individual ablation zones to generate the predicted ablation zone and uses ablation settings that define the size and/or shape of an individual ablation, the one or more settings may comprise one or more of these ablation settings. Thus, in some examples, the one or more settings (which are modified) may define a size of a major and/or minor axes (for an elliptical ablation), as well as (optionally) the offset of a particular ablation zone with respect to an applicator.

Other suitable settings for controlling the generation of the predicted ablation zone will be apparent to the skilled person, and may depend upon the mechanism used to generated the predicted ablation zone from the intra-treatment ablation data.

Step 209 may be performed automatically and/or in response to a manual input.

For manual adjustment of the setting(s), the user/clinician may be able to adjust one or more settings of the ablation system (e.g. the lengths of the major and/or minor axes and/or the offset of an individual ablation zone). This adjustment may be performed by a user interacting with a user interface, e.g. providing a user input. The method may respond to the user adjustment (e.g. via the user input) by updating the predicted ablation zone using the new setting(s), e.g. by regenerating the predicted ablation zone by processing the intra-treatment ablation data using the one or more settings of the ablation system.

Preferably, when a manual adjustment is to take place, step 206 is performed. This enables the user/clinician to observe the effect of their adjustment to the setting(s) of the ablation system and determine whether their adjustment improves or adversely affects the accuracy of the predicted ablation zone (compared to the achieved ablation zone).

To further aid the user/clinician in performing a manual adjustment, an error between the predicted ablation zone and the achieved ablation zone may be calculated and displayed (i.e. steps 207 and 208 may be performed). This provides a quantitative measure of how modifying the setting(s) improves or adversely affects the accuracy of the predicted ablation zone.

Alternatively, the one or more setting(s) of the ablation system may be adjusted automatically. This could be performed using a mathematical optimization approach, such as a multi-dimensional optimization approach. In particular, step 207 may be performed to calculate or generate an error between the predicted ablation zone and the achieved ablation zone, which is then used to execute an optimization approach (e.g. minimize or reduce the error).

In particular examples, a sequence of modifying one or more settings, regenerating the predicted ablation zone and re-determining the error between the predicted ablation zone and the achieved ablation zone may be performed. This sequence can be iteratively repeated using a mathematical optimization approach to modify the one or more settings.

The manual and automated approaches may be combined. For example, an automatic approach to adjusting the one or more settings of the ablation system may be performed, after which the predicted and achieved ablation zones are displayed (e.g. for assessment by a user/clinician), and can then be subject to further modification by the user/clinician.

In both manual and automatic optimization, the method may be adapted to deal gracefully with some of the corner cases of this optimization. For example, individual ablations positioned in the center of a large target, may not affect the outer boundary of the anticipated ablation zone. This should not lead to failures in the automatic optimization, and be clearly communicated to the user in both manual and automatic optimization. In another example, ablation treatments may not necessarily include all types of ablation that could be combined in one treatment. Ablation data for such unused ablations should not be changed.

The process of modifying one of more settings of the ablation system could be performed by any element of the ablation system, e.g. by the ATPS or the ATFS. In preferred examples, the modified settings are provided (or at least made available) to the ATPS to provide improved therapy guidance during subsequent cases.

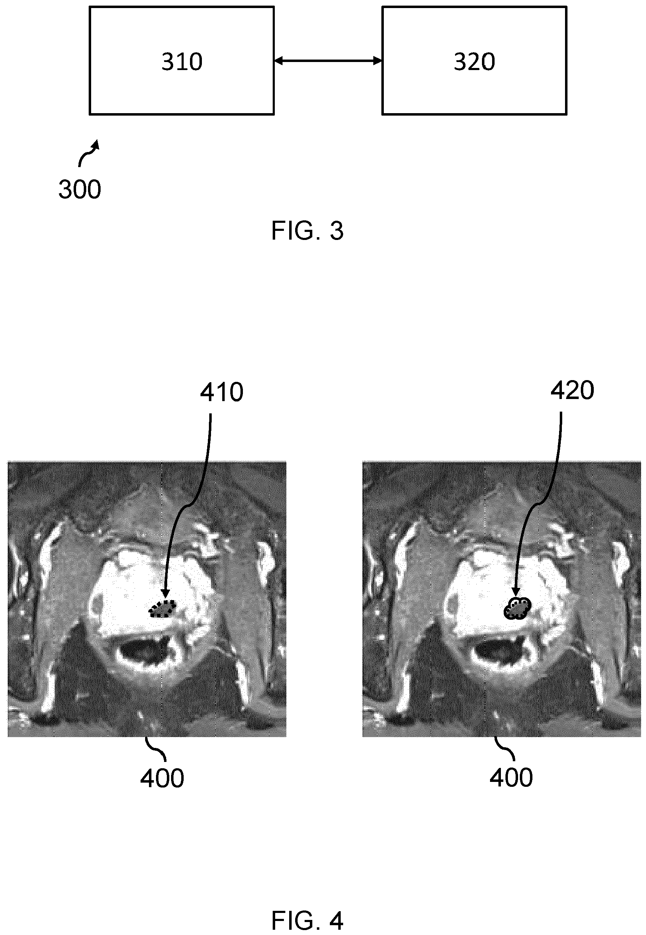

Fig. 3 is a block diagram illustrating an ablation system 300 according to an embodiment of the invention. The ablation system 300 comprises an ablation therapy planning system, ATPS, 310 and an ablation therapy follow-up system, ATFS 320.

The ATPS is adapted to obtain intra-treatment ablation data generated by an ablation system during an ablation treatment, the intra-treatment ablation data providing information on an ablation treatment performed on a subject.

The ATFS is configured to obtain post-treatment ablation data generated after the ablation treatment is performed on the subject, the post-treatment ablation data providing information on the achieved ablation zone of the subject; process the post-treatment ablation data to construct the achieved ablation zone; and register a predicted ablation zone, that predicts the ablation zone produced by the ablation treatment, and the achieved ablation zone with respect to one another to thereby enable assessment of the accuracy of a predicted ablation zone.

Either the ATPS or the ATFS is configured to process the intra-treatment ablation data to construct the predicted ablation zone.

The skilled person would be capable of adapting the ATPS and/or the ATFS to perform any herein described method.

For example, the ATFS preferably comprises a user interface configured to display the predicted ablation zone and the achieved ablation zone, which are registered with respect to one another.

In some examples, where the ATFS is adapted to modify settings used to generate the predicted ablation zone, this information may be passed to the ATPS (which can be used to modify settings of the ATPS, e.g. to improve a display of a predicted ablation zone during an ablation treatment). In such embodiments, the ATPS may be adapted to dynamically generate and display (at a user interface) a predicted ablation zone during the ablation treatment, e.g. based on live intra-treatment ablation data generated by the ATPS.

Fig. 4 is used to illustrate the registration between a predicted ablation zone and an actual ablation zone. For the sake of clarity, these example zones are superimposed over an exemplary MRI zone, to demonstrate a "real-life" correlation between the two zones.

Fig. 4 illustrates an example MRI image 400 (obtained after an ablation treatment has been conducted). There is a well-demarcated demarcated ablation defect with surrounding hyperemia.

In a first instance, only the actual ablation zone 410 is illustrated/delineated (in a dotted line format). This ablation zone is generated using a segmentation process, and is registered/aligned with the MRI image.

In a second instance, the predicted ablation zone 420 is also illustrated (and has been registered with respect to the actual ablation zone (as well as the MRI image). As the two ablation zones are (spatially) registered with respect to one another, the accuracy of the predicted ablation zone can be readily identified and corrected, manually or automatically.

Fig. 4 therefore demonstrates the use and purpose of the present invention, namely to register a predicted and actual ablation zone with respect to one another, so that the accuracy of the predicted ablation zone is made correctable.

Generally speaking, embodiments have been directed towards a concept of assessing and optionally improving the accuracy of the predicted ablation zone, by registering the predicted ablation zone and the achieved ablation with respect to one another.

However, it is also possible to use a planned ablation zone in some embodiments. A planned ablation zone may define a desired area for ablation, e.g. an area of a tumor, and can be used to generation an ablation plan.

Embodiments of the invention may further comprise registering a planned ablation zone to the predicted/achieved ablation zone, to determine an accuracy of the overall ablation treatment with reference to the planned ablation zone. This information could be used to improve the planning of the ablation treatment and/or to identify errors in placement of the ablation applicator(s).

By way of example, if there is only a small error between the predicted ablation zone and the achieved ablation zone, but there is a larger error between the planned ablation zone and the achieved ablation zone, then this is a clear indication that there have been errors in the placement of the ablation applicator(s) during the ablation treatment.

This information could be used to improve the ablation plan, e.g. by identifying areas of the achieved ablation zone that were not reached following the ablation plan.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method for enabling assessment of the accuracy of a predicted ablation zone obtained from intra-treatment data generated by an ablation system during an ablation treatment, the computer-implemented method comprising:
obtaining intra-treatment ablation data generated by an ablation system during an ablation treatment, the intra-treatment ablation data providing information on an ablation treatment performed on a subject,
processing the intra-treatment ablation data to construct a predicted ablation zone that predicts the ablation zone produced by the ablation treatment;
obtaining post-treatment ablation data generated after the ablation treatment is performed on the subject, the post-treatment ablation data providing information on the achieved ablation zone of the subject;
processing the post-treatment ablation data to construct the achieved ablation zone; and
registering the predicted ablation zone and the achieved ablation zone with respect to one another to thereby enable assessment of the accuracy of a predicted ablation zone.

2. The computer-implemented method of claim 1, further comprising determining an error between the predicted ablation zone and the achieved ablation zone.

3. The computer-implemented method of claim 2, further comprising a step of adjusting one or more settings of the ablation system based on the determined error.

4. The computer-implemented method of claim 3, wherein:
the intra-treatment ablation data comprises applicator information identifying at least a position and type of one or more ablation applicators during the ablation treatment;
the step of constructing a predicted ablation zone comprises processing the applicator information and one or more ablation applicator settings to construct the predicted ablation zone, the one or more applicator settings defining the anticipated ablation zone around an individual ablation performed by each ablation applicator based on the applicator information; and
the step of adjusting one or more settings of the ablation system comprises adjusting at least one of the one or more applicator settings.

5. The computer-implemented method of any of claims 3 or 4, further comprising a step of sharing the adjusted settings of the ablation system with one or more other ablation systems.

6. The computer-implemented method of any of claims 1 to 5, wherein the step of registering the predicted ablation zone and the achieved ablation zone comprises registering the intra-treatment ablation data and the post-treatment ablation data with respect to one another, to thereby register the predicted ablation zone and the achieved ablation zone with respect to one another.

7. The computer-implemented method of claim 6, wherein the step of registering the intra-treatment ablation data and the post-treatment ablation data with respect to one another comprises:
obtaining reference ablation data;
registering the intra-treatment ablation data to the reference ablation data; and
registering the post-treatment ablation data to the reference ablation data, thereby indirectly registering the intra-treatment ablation data to the post-treatment ablation data.

8. The computer-implemented method of any of claims 1 to 5, wherein the step of registering the predicted ablation zone and the achieved ablation zone comprises directly processing the predicted ablation zone and the achieved ablation zone to register the two together.

9. The computer-implemented method of any of claims 1 to 8, wherein the post-treatment ablation data comprises one or more medical images of the subject captured after the ablation treatment, the one or more medical images providing visual information on the achieved ablation zone.

10. The computer-implemented method of any of claims 1 to 9, wherein the intra-treatment ablation data comprises one or more medical images captured during the ablation treatment.

11. The computer-implemented method of claim 10, wherein:
the post-treatment ablation data comprises one or more medical images of the subject captured after the ablation treatment, the one or more medical images providing visual information on the achieved ablation zone; and
the step of registering the predicted ablation zone and the achieved ablation zone with respect to one another comprises registering at least one of the medical images captured during the ablation treatment against at least one of the medical images captured after the ablation treatment, to thereby register the predicted ablation zone and the achieved ablation zone with respect to one another.

12. The computer-implemented method of claim 11, wherein the step of registering at least one of the medical images captured during the ablation treatment against at least one of the medical images captured after the ablation treatment comprises:
obtaining a reference medical image;
registering at least one of the medical images captured during the ablation treatment against the reference medical image;
registering at least one of the medical images captured after the ablation treatment against the reference medical image, to thereby indirectly register at least one of the medical images captured during the ablation treatment against at least one of the medical images captured after the medical image.

13. The computer-implemented method of any of claims 1 to 12, further comprising a step of displaying the predicted ablation zone and the achieved ablation zone, wherein the display is based on the registration between the predicted ablation zone and the achieved ablation zone.

14. A computer program product comprising computer program code means which, when executed on a computing device having a processing system, cause the processing system to perform all of the steps of the method according to any of the preceding claims.

15. An ablation system for enabling assessment of the accuracy of a predicted ablation zone obtained from intra-treatment data generated by an ablation system during an ablation treatment, the ablation zone system comprising:
an ablation therapy planning system, ATPS, configured to:
obtain intra-treatment ablation data generated by an ablation system during an ablation treatment, the intra-treatment ablation data providing information on an ablation treatment performed on a subject; and
an ablation therapy follow-up system, ATFS, configured to:
obtain post-treatment ablation data generated after the ablation treatment is performed on the subject, the post-treatment ablation data providing information on the achieved ablation zone of the subject;
process the post-treatment ablation data to construct the achieved ablation zone; and
register a predicted ablation zone, that predicts the ablation zone produced by the ablation treatment, and the achieved ablation zone with respect to one another to thereby enable assessment of the accuracy of a predicted ablation zone,
wherein either the ATPS or the ATFS is configured to process the intra-treatment ablation data to construct the predicted ablation zone.
